(19) Europäisches Patentamt
European Patent Office
Office européen des brevets

(11) **EP 4 692 370 A1**

(12) **EUROPEAN PATENT APPLICATION**
published in accordance with Art. 153(4) EPC

(43) Date of publication:
**11.02.2026 Bulletin 2026/07**

(21) Application number: **24779701.2**

(22) Date of filing: **19.03.2024**

(51) International Patent Classification (IPC):
***C12Q 1/6844** (2018.01)* ***C08F 20/36** (2006.01)*
***C12Q 1/48** (2006.01)*

(52) Cooperative Patent Classification (CPC):
**C08F 20/36; C12Q 1/48; C12Q 1/6844**

(86) International application number:
**PCT/JP2024/010666**

(87) International publication number:
**WO 2024/203576 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.03.2023 JP 2023055637**

(71) Applicant: **NOF Corporation**
**Shibuya-ku**
**Tokyo 150-6019 (JP)**

(72) Inventors:
- **KOHATSU, Haruki**
  **Kawasaki-shi, Kanagawa 210-0865 (JP)**
- **SUZUKI, Hirotaka**
  **Kawasaki-shi, Kanagawa 210-0865 (JP)**
- **MATSUDA, Masaru**
  **Kawasaki-shi, Kanagawa 210-0865 (JP)**

(74) Representative: **J A Kemp LLP**
**80 Turnmill Street**
**London EC1M 5QU (GB)**

# (54) FREEZE-THAWING PROTECTIVE AGENT FOR NUCLEIC ACID AMPLIFICATION COMPOSITION AND NUCLEIC ACID AMPLIFICATION METHOD USING SAME

(57) The present invention provides a freeze-thawing protective agent for a composition for nucleic acid amplification, which is a polymer containing constitutional units derived from a monomer represented by the following formula (1) (the definitions of symbols in the formula are as described in the specification).

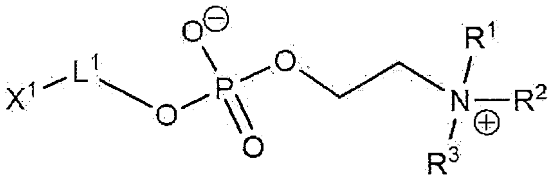

(1)

EP 4 692 370 A1

## Description

[Technical Field]

The present invention relates to a freeze-thawing protective agent for a composition for nucleic acid amplification and a nucleic acid amplification method using same.

[Background Art]

Nucleic acid amplification method is known as a reaction that uses a nucleic acid as a template to newly synthesize a nucleic acid complementary to the nucleic acid. In order to perform a nucleic acid amplification reaction using this nucleic acid amplification method, multiple nucleic acid amplification reagents, such as oligonucleotides called primers and enzymes, are required in addition to the nucleic acid to be the template. Since these nucleic acid amplification reagents are unstable at room temperature or under refrigerated conditions, they are stored under frozen conditions (e.g., -80°C to -20°C) and thawed before use, and such freeze-thawing is repeated. However, it is known that freezing and thawing proteins changes their higher-order structures and causes deactivation.

To prevent denaturation of protein due to freeze-thawing, it is known that sugars, glycerin, albumin, and the like are added to compositions for nucleic acid amplification. However, these substances must be added at extremely high concentrations, and compositions for nucleic acid amplification containing these substances at high concentrations must have the sugars removed by dialysis or the like after thawing (e.g., Patent Literatures 1 and 2). As another method, dimethyl sulfoxide (hereinafter referred to as "DMSO") is used as a protective agent. However, DMSO is known to show toxicity to proteins and cell metabolism.

[Citation List]

[Patent Literature]

[Patent Literature 1]
JP 2008-203269 A
[Patent Literature 2]
JP 2017-137328 A

[Summary of Invention]

[Technical Problem]

Compositions obtained by adding sugars, glycerin, albumin, DMSO, etc. as a freeze-thawing protective agent to compositions for nucleic acid amplification are less susceptible to deactivation due to freeze-thawing than compositions without addition of these agents. However, the aforementioned sugars and the like have problems, such as the need to add them at high concentrations, the need for a removal operation before use, and the possibility of affecting the properties of the target substances.

The present invention has been made in view of the above-mentioned situation, and the object of the present invention is to provide a freeze-thawing protective agent for compositions for nucleic acid amplification that can reduce, at a relatively low concentration, deactivation of compositions for nucleic acid amplification due to freeze-thawing.

[Solution to Problem]

The present inventors have conducted intensive studies and found that a polymer containing constitutional units derived from a monomer represented by the following formula (1) can be used as a freeze-thawing protective agent for a composition for nucleic acid amplification that can achieve the above-mentioned object. Explanation based on this finding is as follows.

[1] A freeze-thawing protective agent for a composition for nucleic acid amplification, which is a polymer comprising constitutional units derived from a monomer represented by the formula (1):

[Chem. 1]

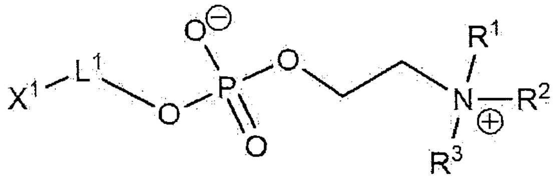

(1)

wherein

$X^1$ is a (meth)acryloyloxy group or a (meth)acryloylamino group,
$L^1$ is an alkylene group having 2 to 4 carbon atoms and optionally having one hydroxy group, or an alkyleneoxyalkylene group having 2 to 4 carbon atoms, and
$R^1$ to $R^3$ are each independently an alkyl group having 1 to 3 carbon atoms.

[2] The freeze-thawing protective agent of the aforementioned [1], wherein the monomer represented by the aforementioned formula (1) is a monomer in which $X^1$ is a (meth)acryloyloxy group, $L^1$ is $-C_2H_4-$ or $-C_2H_4-O-C_2H_4-$, and $R^1$ to $R^3$ are each a methyl group, preferably 2-(meth)acryloyloxyethyl phosphorylcholine, more preferably 2-methacryloyloxyethyl phosphorylcholine.
[3] The freeze-thawing protective agent of the aforementioned [1] or [2], wherein the aforementioned polymer is a copolymer further comprising constitutional units derived from a monomer represented by the formula (2):

[Chem. 2]

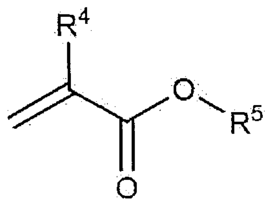

(2)

wherein

$R^4$ is a hydrogen atom or a methyl group, and
$R^5$ is a hydrogen atom, an alkyl group having 1 to 20 carbon atoms, $-(OCH_2CH_2)_nOR^6$ group (wherein n is a number from 1 to 10, and $R^6$ is a hydrogen atom, a methyl group, or an ethyl group), or an aralkyl group having 7 to 12 carbon atoms.

[4] The freeze-thawing protective agent of the aforementioned [3], wherein the aforementioned copolymer is a copolymer consisting of constitutional units derived from a monomer represented by the aforementioned formula (1) and constitutional units derived from a monomer represented by the aforementioned formula (2), and $R^5$ is a hydrogen atom or an alkyl group having 1 to 20 carbon atoms.
[5] The freeze-thawing protective agent of the aforementioned [4], wherein the monomer represented by the aforementioned formula (2) is at least one selected from the group consisting of (meth)acrylic acid, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, and stearyl (meth)acrylate, preferably at least one selected from the group consisting of (meth)acrylic acid, pentadecyl (meth)acrylate, cetyl (meth)acrylate, heptadecyl (meth)acrylate, and stearyl (meth)acrylate, more preferably at least one selected from the group consisting of (meth)acrylic acid and stearyl (meth)acrylate, further preferably at least one selected from the group consisting of methacrylic acid and stearyl methacrylate.
[6] The freeze-thawing protective agent of the aforementioned [4] or [5], wherein the constitutional units derived from the monomer represented by the aforementioned formula (1) have a quantitative ratio of 5 to 95 mol, preferably 10 to 95 mol, more preferably 15 to 95 mol, further preferably 20 to 90 mol, relative to the total 100 mol of the constitutional units derived from the monomer represented by the aforementioned formula (1) and the constitutional units derived

from the monomer represented by the aforementioned formula (2).

[7] The freeze-thawing protective agent of any one of the aforementioned [4] to [6], wherein the aforementioned copolymer has a weight average molecular weight of 10,000 to 2,000,000, preferably 20,000 to 1,500,000, more preferably 30,000 to 1,000,000.

[8] The freeze-thawing protective agent of the aforementioned [3], wherein the aforementioned copolymer is a copolymer consisting of the constitutional units derived from the monomer represented by the aforementioned formula (1) and the constitutional units derived from the monomer represented by the aforementioned formula (2), and $R^5$ is a $-(OCH_2CH_2)_nOR^6$ group (wherein n is a number from 1 to 10, preferably a number from 5 to 10, more preferably a number from 8 to 10, and $R^6$ is a hydrogen atom, a methyl group, or an ethyl group).

[9] The freeze-thawing protective agent of the aforementioned [8], wherein the monomer represented by the aforementioned formula (2) is at least one selected from the group consisting of methoxypolyethylene glycol (meth)acrylate, and ethoxypolyethylene glycol (meth)acrylate, preferably methoxypolyethylene glycol methacrylate.

[10] The freeze-thawing protective agent of the aforementioned [8] or [9], wherein the constitutional units derived from the monomer represented by the aforementioned formula (1) have a quantitative ratio of 40 to 95 mol, preferably 60 to 95 mol, more preferably 80 to 95 mol, relative to the total 100 mol of the constitutional units derived from the monomer represented by the aforementioned formula (1) and the constitutional units derived from the monomer represented by the aforementioned formula (2).

[11] The freeze-thawing protective agent of any one of the aforementioned [8] to [10], wherein the aforementioned copolymer has a weight average molecular weight of 100,000 to 1,000,000, preferably 200,000 to 1,000,000, more preferably 300,000 to 800,000.

[12] The freeze-thawing protective agent of the aforementioned [3], wherein the aforementioned copolymer is a copolymer consisting of the constitutional units derived from the monomer represented by the aforementioned formula (1) and the constitutional units derived from the monomer represented by the aforementioned formula (2), and $R^5$ is an aralkyl group having 7 to 12 carbon atoms.

[13] The freeze-thawing protective agent of the aforementioned [12], wherein the monomer represented by the aforementioned formula (2) is at least one selected from the group consisting of benzyl (meth)acrylate, 2-phenylethyl (meth)acrylate, and 3-phenylpropyl (meth)acrylate, preferably benzyl methacrylate.

[14] The freeze-thawing protective agent of the aforementioned [12] or [13], wherein the constitutional units derived from the monomer represented by the aforementioned formula (1) have a quantitative ratio of 40 to 95 mol, preferably 60 to 95 mol, more preferably 70 to 90 mol, relative to the total 100 mol of the constitutional units derived from the monomer represented by the aforementioned formula (1) and the constitutional units derived from the monomer represented by the aforementioned formula (2).

[15] The freeze-thawing protective agent of any one of the aforementioned [12] to [14], wherein the aforementioned copolymer has a weight average molecular weight of 50,000 to 1,000,000, preferably 100,000 to 800,000, more preferably 100,000 to 500,000.

[16] A composition for nucleic acid amplification, comprising the composition of any one of the aforementioned [1] to [15] and water.

[17] The composition of the aforementioned [16], further comprising a polymerase.

[18] The composition of the aforementioned [16] or [17], further comprising a primer.

[19] The composition of any one of the aforementioned [16] to [18], wherein the aforementioned freeze-thawing protective agent in the aforementioned composition for nucleic acid amplification has a concentration of 0.00001 to 10 w/v%, preferably 0.001 to 1 w/v%, more preferably 0.01 to 0.5 w/v%.

[20] The composition of any one of the aforementioned [16] to [19], which is frozen.

[21] A method for amplifying a nucleic acid, comprising preparing a reaction solution for nucleic acid amplification by mixing the composition of any one of the aforementioned [16] to [19] and a sample comprising the nucleic acid to be amplified.

[22] A method for amplifying a nucleic acid, comprising preparing a reaction solution for nucleic acid amplification by mixing a composition for nucleic acid amplification obtained by thawing the frozen composition for nucleic acid amplification of the aforementioned [20] and a sample comprising the nucleic acid to be amplified.

[Advantageous Effects of Invention]

The freeze-thawing protective agent for a composition for nucleic acid amplification of the present invention can reduce deactivation of a composition for nucleic acid amplification due to freeze-thawing by adding the agent to the composition for nucleic acid amplification.

[Description of Embodiments]

The present invention is described in detail below.

When stepwise numerical ranges are described in the present specification, the lower limit and the upper limit of each numerical range can be combined. In the case of, for example, "preferably 10 to 100, more preferably 20 to 90", the preferred lower limit 10 can be combined with the more preferred upper limit 90 (i.e., the numerical range of "10 to 90" is also within the range of the present specification).

[Freeze-thawing protective agent]

The present invention provides a freeze-thawing protective agent for a composition for nucleic acid amplification (hereinafter sometimes referred to as "the protective agent of the present invention"), which is a polymer containing constitutional units derived from a monomer represented by the formula (1) (hereinafter sometimes referred to as "the polymer of the present invention").

In the present specification, the "composition for nucleic acid amplification" means a composition used in the nucleic acid amplification method.

In the present specification, the "freeze-thawing protective agent for a composition for nucleic acid amplification" means an additive used for reducing the deactivation of the composition for nucleic acid amplification due to freeze-thawing.

The polymer of the present invention is a polymer containing constitutional units derived from a monomer represented by the formula (1):

[Chem. 3]

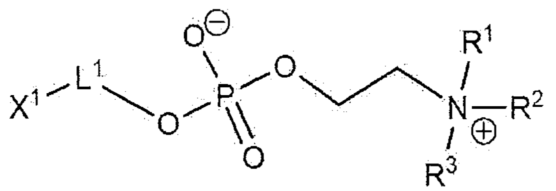 (1)

wherein

$X^1$ is a (meth)acryloyloxy group or a (meth)acryloylamino group,
$L^1$ is an alkylene group having 2 to 4 carbon atoms and optionally having one hydroxy group, or an alkyleneoxyalkylene group having 2 to 4 carbon atoms, and
$R^1$ to $R^3$ are each independently an alkyl group having 1 to 3 carbon atoms. Only one kind of the polymer of the present invention may be used, or two or more kinds thereof may be used in combination.

Hereinafter, the "monomer represented by the formula (1)" is sometimes abbreviated as "monomer (1)". The "monomers represented by other formulas" are also sometimes abbreviated in the same manner as "the monomer represented by the formula (1)".

The "constitutional unit derived from monomer (1)" is sometimes abbreviated as "constitutional unit (1)". The "constitutional unit derived from other monomer" is sometimes abbreviated in the same manner as the "constitutional unit derived from monomer (1)".

The constitutional unit derived from monomer (1) means a constitutional unit having a structure formed by the reaction of a carbon-carbon double bond of the (meth)acryloyl group contained in monomer (1). The constitutional units derived from other monomers means the same as the constitutional unit derived from monomer (1).

Only one kind of monomer (1) may be used, or two or more kinds thereof may be used in combination. That is, the polymer of the present invention may be a homopolymer consisting of one kind of constitutional units (1), or a copolymer containing two or more kinds of constitutional units (1). The aforementioned copolymer may be a random copolymer, a block copolymer, or a copolymer containing both a random portion and a block portion.

The groups in the formula (1) are described in order below. In the formula (1), $X^1$ is a (meth)acryloyloxy group (i.e., $CH_2=CR-CO-O-$, R: hydrogen atom or a methyl group) or a (meth)acryloylamino group (i.e., $CH_2=CR-CO-NH-$, R: hydrogen atom or a methyl group). From the aspect of the availability of the starting materials, $X^1$ is preferably a (meth) acryloyloxy group, more preferably a methacryloyloxy group.

In the formula (1), $L^1$ is an alkylene group having 2 to 4 carbon atoms and optionally having one hydroxy group, or

an alkyleneoxyalkylene group having 2 to 4 carbon atoms. The aforementioned alkylene group may be linear or branched chain. Examples of the alkylene group having 2 to 4 carbon atoms and optionally having one hydroxy group include $-C_2H_4-$. Examples of the alkyleneoxyalkylene group having 2 to 4 carbon atoms include $-C_2H_4-O-C_2H_4-$. From the aspect of the availability of the starting materials, $L^1$ is preferably $-C_2H_4-$ or $-C_2H_4-O-C_2H_4-$, more preferably $-C_2H_4-$ (i.e., ethylene group).

In the formula (1), $R^1$ to $R^3$ are each independently an alkyl group having 1 to 3 carbon atoms. The aforementioned alkyl group may be linear or branched chain. Examples of the alkyl group having 1 to 3 carbon atoms include methyl group, ethyl group, propyl group, and the like. From the aspect of the availability of the starting materials, $R^1$ to $R^3$ are preferably methyl groups.

Preferred monomer (1) is a monomer in which $X^1$ is a (meth) acryloyloxy group, $L^1$ is $-C_2H_4-$ or $-C_2H_4-O-C_2H_4-$, and $R^1$ to $R^3$ are methyl groups. More preferred monomer (1) is a monomer in which $X^1$ is a (meth)acryloyloxy group, $L^1$ is an ethylene group, and $R^1$ to $R^3$ are methyl groups (i.e., 2-(meth)acryloyloxyethyl phosphorylcholine). Further preferred monomer (1) is 2-methacryloyloxyethyl phosphorylcholine. A commercially available product can be used for monomer (1).

In the present specification, "2-(meth)acryloyloxyethyl phosphorylcholine" basically means "2-acryloyloxyethyl phosphorylcholine or 2-methacryloyloxyethyl phosphorylcholine". When a plurality of the 2-(meth)acryloyloxyethyl phosphorylcholine may be present, the "2-(meth)acryloyloxyethyl phosphorylcholine" means "2-acryloyloxyethyl phosphorylcholine and/or 2-methacryloyloxyethyl phosphorylcholine". Other terms similar to the "2-(meth)acryloyloxyethyl phosphorylcholine" also mean the same as the "(2-(meth)acryloyloxyethyl phosphorylcholine".

One embodiment of the polymer of the present invention is a polymer consisting of constitutional units (1) (hereinafter sometimes abbreviated as "polymer (1)"). In the present specification, the "polymer consisting of constitutional units (1)" means a polymer in which all constitutional units (repeating units) in the polymer chain consists of constitutional units (1). Expressions similar to the "polymer consisting of constitutional units (1)" means the same as the "polymer consisting of constitutional units (1)". Polymer (1) may be a homopolymer consisting of one kind of constitutional units (1), or a copolymer consisting of two or more kinds of constitutional units (1), and is preferably a copolymer consisting of two or more kinds of constitutional units (1).

While the weight average molecular weight of the polymer (1) is not particularly limited, it is preferably 20,000 to 2,000,000, more preferably 100,000 to 1,500,000, further preferably 500,000 to 1,500,000. The weight average molecular weight can be determined based on polyethylene glycol by gel filtration chromatography using, for example, EcoSEC system (manufactured by Tosoh Corporation) or the like.

The polymer of the present invention may further contain, in addition to constitutional units (1), constitutional units derived from a monomer represented by the formula (2):

[Chem. 4]

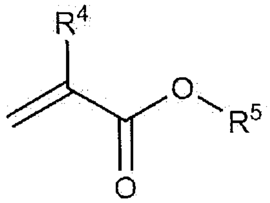

(2)

wherein

$R^4$ is a hydrogen atom or a methyl group, and
$R^5$ is a hydrogen atom, an alkyl group having 1 to 20 carbon atoms, a $-(OCH_2CH_2)_nOR^6$ group (wherein n is a number from 1 to 10, and $R^6$ is a hydrogen atom, a methyl group, or an ethyl group), or an aralkyl group having 7 to 12 carbon atoms).

The groups in the formula (2) are described in order below. In the formula (2), $R^4$ is a hydrogen atom or a methyl group. From the aspect of the preservation stability of the polymer, $R^4$ is preferably a methyl group.

In the formula (2), $R^5$ is a hydrogen atom, an alkyl group having 1 to 20 carbon atoms, $-(OCH_2CH_2)_nOR^6$ group, or an aralkyl group having 7 to 12 carbon atoms.

A monomer in which $R^5$ in the formula (2) is a hydrogen atom or an alkyl group having 1 to 20 carbon atoms (hereinafter sometimes to be abbreviated as "monomer (2a)") is explained. The aforementioned alkyl group may be linear or branched chain. In monomer (2a), $R^5$ is preferably a hydrogen atom or an alkyl group having 10 to 20 carbon atoms, more preferably a hydrogen atom or an alkyl group having 15 to 19 carbon atoms, further preferably a hydrogen atom or a

linear alkyl group having 17 to 19 carbon atoms.

Specific examples of monomer (2a) include (meth)acrylic acid, methyl (meth)acrylate, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, stearyl (meth)acrylate, and the like. Only one kind of monomer (2a) may be used, or two or more kinds thereof may be used in combination. A commercially available product can be used for monomer (2a).

Among the aforementioned specific examples of monomer (2a),

(i) (meth)acrylic acid, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, and stearyl (meth)acrylate are preferred,
(ii) (meth)acrylic acid, pentadecyl (meth)acrylate, cetyl (meth)acrylate, heptadecyl (meth)acrylate, and stearyl (meth) acrylate are more preferred,
(iii) (meth)acrylic acid and stearyl (meth)acrylate are further preferred,
(iv) methacrylic acid and stearyl methacrylate are particularly preferred.

One embodiment of the polymer of the present invention is a copolymer consisting of constitutional units (1) and constitutional units (2a) (hereinafter sometimes to be abbreviated as "copolymer (1-2a)"). Only one kind each of monomer (1) and monomer (2a) may be used, or two or more kinds thereof may be used in combination. That is, copolymer (1-2a) may be a copolymer containing one or more kinds of constitutional units (1) and one or more kinds of constitutional units (2a). The aforementioned copolymer may be a random copolymer, a block copolymer, or a copolymer containing both a random portion and a block portion.

In copolymer (1-2a), the quantitative ratio of constitutional units (1) (i.e., quantitative ratio of monomers (1) used in polymerization) is preferably 5 to 95 mol, more preferably 10 to 95 mol, further preferably 15 to 95 mol, particularly preferably 20 to 90 mol, and the quantitative ratio of constitutional units (2a) (i.e., quantitative ratio of monomers (2a) used in polymerization) is preferably 5 to 95 mol, more preferably 5 to 90 mol, further preferably 5 to 85 mol, particularly preferably 10 to 80 mol, each relative to the total 100 mol of the constitutional units (1) and constitutional units (2a) (i.e., total 100 mol of monomers (1) and monomers (2a) used in polymerization).

While the weight average molecular weight of copolymer (1-2a) is not particularly limited, it is preferably 10,000 to 2,000,000, more preferably 20,000 to 1,500,000, further preferably 30,000 to 1,000,000. The weight average molecular weight can be determined based on polyethylene glycol by gel filtration chromatography using, for example, EcoSEC system (manufactured by Tosoh Corporation) or the like.

A monomer in which $R^5$ in the formula (2) is a $-(OCH_2CH_2)_nOR^6$ group (wherein n is a number from 1 to 10, and $R^6$ is a hydrogen atom, a methyl group, or an ethyl group) (hereinafter sometimes to be abbreviated as "monomer (2b)") is explained.

$R^6$ is preferably a methyl group or an ethyl group, more preferably a methyl group.

In the $-(OCH_2CH_2)_nOR^6$ group, n is a number from 1 to 10, preferably a number from 5 to 10, more preferably a number from 8 to 10.

Specific examples of monomer (2b) include polyethylene glycol mono(meth)acrylate, methoxypolyethylene glycol (meth)acrylate, ethoxypolyethylene glycol (meth)acrylate, and the like. Only one kind of monomer (2b) may be used, or two or more kinds thereof may be used in combination. From the aspect of the preservation stability of the polymer, methoxypolyethylene glycol (meth)acrylate and ethoxypolyethylene glycol (meth)acrylate are preferred, and methoxypolyethylene glycol methacrylate is more preferred. As monomer (2b), a commercially available product can be used.

One embodiment of the polymer of the present invention is a copolymer consisting of constitutional units (1) and constitutional units (2b) (hereinafter sometimes to be abbreviated as "copolymer (1-2b)"). Only one kind each of monomer (1) and monomer (2b) may be used, or two or more kinds thereof may be used in combination. That is, copolymer (1-2b) may be a copolymer containing one or more kinds of constitutional units (1) and one or more kinds of constitutional units (2b). The aforementioned copolymer may be a random copolymer, a block copolymer, or a copolymer containing both a random portion and a block portion.

In copolymer (1-2b), the quantitative ratio of constitutional units (1) (i.e., quantitative ratio of monomers (1) used in polymerization) is preferably 40 to 95 mol, more preferably 60 to 95 mol, further preferably 80 to 95 mol, and the quantitative ratio of constitutional units (2b) (i.e., quantitative ratio of monomers (2b) used in polymerization) is preferably 5 to 60 mol, more preferably 5 to 40 mol, further preferably 5 to 20 mol, each relative to the total 100 mol of the constitutional units (1) and constitutional units (2b) (i.e., total 100 mol of monomers (1) and monomers (2b) used in polymerization).

While the weight average molecular weight of the polymer (1-2b) is not particularly limited, it is preferably 100,000 to 1,000,000, more preferably 200,000 to 1,000,000, further preferably 300,000 to 800,000.

A monomer in which $R^5$ in the formula (2) is an aralkyl group having 7 to 12 carbon atoms (hereinafter sometimes

to be abbreviated as "monomer (2c)") is explained. The alkyl group in the aralkyl group having 7 to 12 carbon atoms may be linear or branched chain. Examples of the the aralkyl group having 7 to 12 carbon atoms include benzyl group, 2-phenylethyl group, 3-phenylpropyl group, 4-phenylbutyl group, 5-phenylpentyl group, and the like. The carbon number of the aralkyl group is more preferably 7 to 9.

Specific examples of monomer (2c) include benzyl (meth)acrylate, 2-phenylethyl (meth)acrylate, 3-phenylpropyl (meth)acrylate, 4-phenylbutyl (meth)acrylate, 5-phenylpentyl (meth)acrylate and the like. From the aspect of the preservation stability of the polymer, benzyl (meth)acrylate, 2-phenylethyl (meth)acrylate, and 3-phenylpropyl (meth)acrylate are preferred, and benzyl methacrylate is more preferred. Only one kind of monomer (2c) may be used, or two or more kinds thereof may be used in combination. As monomer (2c), a commercially available product can be used.

One embodiment of the polymer of the present invention is a copolymer consisting of constitutional units (1) and constitutional units (2c) (hereinafter sometimes to be abbreviated as "copolymer (1-2c)"). Only one kind each of monomer (1) and monomer (2c) may be used, or two or more kinds thereof may be used in combination. That is, copolymer (1-2c) may be a copolymer containing one or more kinds of constitutional units (1) and one or more kinds of constitutional units (2c). The aforementioned copolymer may be a random copolymer, a block copolymer, or a copolymer containing both a random portion and a block portion.

In copolymer (1-2c), the quantitative ratio of constitutional units (1) (i.e., quantitative ratio of monomers (1) used in polymerization) is preferably 40 to 95 mol, more preferably 60 to 95 mol, further preferably 70 to 90 mol, and the quantitative ratio of constitutional units (2c) (i.e., quantitative ratio of monomers (2c) used in polymerization) is preferably 5 to 60 mol, more preferably 5 to 40 mol, further preferably 10 to 30 mol, each relative to the total 100 mol of the constitutional units (1) and constitutional units (2c) (i.e., total 100 mol of monomers (1) and monomers (2c) used in polymerization).

While the weight average molecular weight of the polymer (1-2c) is not particularly limited, it is preferably 50,000 to 1,000,000, more preferably 100,000 to 800,000, further preferably 100,000 to 500,000.

The polymer of the present invention may contain, as long as the effect of the present invention is not impaired, other constitutional units derived from other monomers different from monomer (1) and monomer (2) (i.e., monomers (2a) to (2c)). Only one kind of other monomer may be used, or two or more kinds thereof may be used in combination. Other monomer is not particularly limited, and examples thereof include glycerol methacrylate, isobornyl (meth)acrylate, and the like. The amount of other constitutional units in the polymer of the present invention is preferably not more than 20 mol% with respect to the total constitutional units. More preferably, the polymer of the present invention does not contain other constitutional units.

The polymer of the present invention can be produced by known methods (e.g., the method described in WO 2018/216628).

The amount of the protective agent of the present invention (that is, the polymer of the present invention) is determined by the concentration in the composition for nucleic acid amplification described below. The concentration of the protective agent of the present invention in the aforementioned composition is preferably 0.00001 to 10 w/v%, more preferably 0.001 to 1 w/v%, further preferably 0.01 to 0.5 w/v%, from the aspect of the protection effect. When two or more kinds of protective agents are used, the aforementioned concentration means the total concentration of the two or more kinds of protective agents. When two or more kinds of the following other components are used, the concentrations described for such other components also refer to the total of the concentrations of the two or more components.

[Composition for nucleic acid amplification]

The present invention also provides a composition for nucleic acid amplification containing the protective agent of the present invention and water (hereinafter sometimes to be indicated as "the composition of the present invention"). The protective agent of the present invention is as described above.

Examples of the nucleic acid amplification method using the composition of the present invention include polymerase chain reaction (PCR) method, loop mediated isothermal amplification (LAMP) method, transcription mediated amplification (TMA) method, isothermal and chimeric primer-initiated amplification of nucleic acids (ICAN) method, strand displacement amplification (SDA) method, ligase chain reaction (LCR) method, nucleic acid sequence-based amplification (NASBA) method and the like.

The nucleic acid amplification method is preferably a polymerase chain reaction method (PCR method), more preferably a quantitative polymerase chain reaction method. That is, the composition of the present invention is more preferably used in the quantitative polymerase chain reaction method. In the present specification, the "polymerase chain reaction method (PCR method)" includes not only a typical PCR method using DNA as the target nucleic acid, but also reverse transcription polymerase chain reaction (RT-PCR) method using RNA as the target nucleic acid. In the present specification, moreover, the "quantitative polymerase chain reaction method" includes not only a typical quantitative polymerase chain reaction method using DNA as the target nucleic acid, but also a quantitative reverse transcription polymerase chain reaction method using RNA as the target nucleic acid.

[Other component]

The composition of the present invention contains components other than the polymer of the present invention (hereinafter sometimes referred to as "other component"). As other component, known components used for nucleic acid amplification method represented by PCR method can be used. Examples of other component include polymerase, primer, buffer, substrate, fluorescent probe, passive reference, salt, surfactant, protein, nucleic acid, and the like. Only one kind of other component may be used, or two or more kinds thereof may be used in combination.

As the polymerase, known DNA polymerase can be used. From the aspect of heat resistance, enzymes derived from thermophilic bacteria, thermophilic archaea, hyperthermophilic bacteria, or hyperthermophilic archaea, and mutant enzymes thereof are preferred. A DNA polymerase is appropriately selected from a DNA-dependent DNA polymerase, an RNA-dependent DNA polymerase, or an enzyme having both functions, depending on the purpose of nucleic acid amplification. Moreover, whether to use a DNA polymerase having nuclease activity or a DNA polymerase having no nuclease activity is appropriately determined.

The composition of the present invention preferably contains a polymerase. When the composition of the present invention contains a polymerase, the content thereof (i.e., the amount (U) of polymerase per 1 μL of the composition of the present invention) is preferably 0.01 to 0.1 U/μL.

The primer is not particularly limited. Examples thereof include oligonucleotides with a length of about 15 to 30 bases designed and prepared by known methods. The primer may also be modified as appropriate with a fluorescence dye and the like such as fluorescein (FAM) and the like. Only one kind of primer may be used, or two kinds thereof may be used in a pair. A plurality of primers may also be used to simultaneously amplify plural regions.

The composition of the present invention preferably contains a primer. When the composition of the present invention contains a primer, the content thereof (i.e., the amount (pmol) of primer per 1 μL of the composition of the present invention) is preferably 0.1 to 1 pmol/μL.

Buffer is not particularly limited. Examples thereof include buffers with a pH adjusted to about 6 to 9, more preferably about 7 to 8, by mixing an organic base such as tris(hydroxymethyl)aminomethane, Tricine, Bicine, and the like and an acid such as sulfuric acid, hydrochloric acid, acetic acid, phosphoric acid, and the like. The buffer desirably contains a magnesium salt (e.g., magnesium chloride) and/or a manganese salt (e.g., manganese acetate) as appropriate. The buffer may further contain a salt such as potassium chloride, ammonium sulfate, and the like. The buffer may further contain a water-soluble organic solvent such as dimethyl sulfoxide, dimethylformamide, formamide, glycerin, and the like. The buffer may further contain a surfactant such as polyoxyethylene sorbitan fatty acid ester, polyoxyethylene alkylphenyl ether, and the like. The buffer may further contain a protein such as bovine serum albumin and the like.

While the substrate is not particularly limited, examples thereof include a mixture (dNTPs) of deoxyadenosine triphosphate (dATP), deoxythymidine triphosphate (dTTP), deoxyguanosine triphosphate (dGTP), and deoxythymidine triphosphate (dCTP). It is also possible to partially and/or entirely substitute dTTP with deoxyuridine triphosphate (dUTP).

The fluorescent probe is not particularly limited, and examples thereof include TaqMan™ probe.

Passive reference may be selected appropriately according to the purpose of nucleic acid amplification. Examples of the passive reference include ROX™ Dye and the like.

Examples the salt include, but are not limited to, salts of an organic base such as tris(hydroxymethyl)aminomethane, tricine, bicine, and the like and, with an acid such as sulfuric acid, hydrochloric acid, acetic acid, phosphoric acid, and the like. Also, magnesium salts (e.g., magnesium chloride), manganese salts (e.g., manganese acetate), potassium chloride, and ammonium sulfate may also be used as the salt.

Examples the surfactant include, but are not limited to, polyoxyethylene sorbitan fatty acid ester and polyoxyethylene alkylphenyl ether.

Examples the protein include, but are not limited to, bovine serum albumin.

As the nucleic acid, any DNA and/or RNA may be used, for example, as an exogenous control gene, in addition to the aforementioned primers and fluorescent probes. Here, the nucleic acid may be synthesized in vitro, or may be prepared from cells, microorganisms, viruses, or the like by known methods. Here, the cells, microorganisms, viruses, and the like may be those collected from human, animals, or plants in the natural world or environment, or may be those obtained by isolation and culture.

The composition of the present invention may further contain oil such as mineral oil and the like; solid phase support such as glass beads, magnetic beads, and the like.

In addition, kit-type products in which a plurality of the above-mentioned components are combined, or master-mix (sometimes called primer mix, pre-mix, etc.) type products in which those components are mixed in advance may also be used.

Since the composition of the present invention contains the protective agent of the present invention, deactivation due to freeze-thawing is suppressed. Therefore, the composition of the present invention is preferably stored frozen. Such frozen composition of the present invention is also within the scope of the present invention.

[Nucleic acid amplification method of the present invention]

The present invention also provides a method for amplifying a nucleic acid, which includes preparing a reaction solution for nucleic acid amplification by mixing the composition of the present invention and a sample comprising the nucleic acid to be amplified. In addition, the present invention also provides a nucleic acid amplification method, which includes mixing a composition for nucleic acid amplification obtained by thawing the composition of the present invention that has been frozen (hereinafter sometimes referred to as the "composition after freeze-thawing") and a sample containing the nucleic acid to be amplified, to prepare a reaction solution for nucleic acid amplification. The aforementioned reaction solution can be produced, for example, by mixing a sample containing the nucleic acid to be amplified and water (i.e., nucleic acid aqueous solution) with the composition of the present invention or the composition after freeze-thawing. The composition of the present invention in the nucleic acid amplification method of the present invention is as described above.

The nucleic acid to be amplified may be any of DNA and RNA.

The nucleic acid amplification method is as described above. The nucleic acid amplification method of the present invention is preferably a polymerase chain reaction method (PCR method), more preferably a quantify polymerase chain reaction method (qPCR method). The nucleic acid amplification method (particularly PCR method) is well known to those of ordinary skill in the art, and those of ordinary skill in the art can appropriately perform the method.

[Example]

The present invention is explained in more detail in the following by referring to Examples and the like, which are not to be construed as limitative.

[Synthetic Example 1]

2-Methacryloyloxyethyl phosphorylcholine (hereinafter referred to as "MPC") (6.0 g) as monomer (1), and methacrylic acid (hereinafter referred to as "MAc") (4.0 g) as monomer (2a) (monomer (1)/monomer (2)=30/70 (molar ratio)) were weighed into a glass flask for polymerization, purified water (90.0 g) was added to dissolve monomer (1) and monomer (2a), and PEROYL SA (manufactured by NOF CORPORATION, hereinafter referred to as "PRSA") (0.78 g) was added to the obtained solution. After the inside of the reaction container was sufficiently replaced with nitrogen, polymerization was performed by heating at 70°C for 6 hr while stirring. The obtained reaction solution was ice-cooled and added dropwise to acetone to precipitate a polymer. The precipitate was collected by filtration, washed with acetone, and vacuum dried to give a homopolymer (hereinafter to be indicated as "polymer 1") as a white powder. The weight average molecular weight of polymer 1 was 680,000 based on polyethylene glycol as measured by GPC under the below-mentioned conditions.

[Synthetic Example 2]

MPC (25.3 g) as monomer (1) and methoxypolyethylene glycol methacrylate (BLEMMER PME, manufactured by NOF CORPORATION) (number average molecular weight of monomer (2b): about 500, n in $-(OCH_2CH_2)_nOR^6$ group: about 9, hereinafter referred to as "PEGMA") (4.7 g) as monomer (2b) (monomer (1)/monomer (2b)=90/10(molar ratio)) were weighed into a glass flask for polymerization, purified water (70.0 g) was added to dissolve monomers (1) and (2b), and PRSA (0.23 g) was added to the obtained solution. Thereafter, in the same manner as in Synthetic Example 1, a copolymer (hereinafter referred to as "polymer 2") was obtained. The weight average molecular weight of polymer 2 was 501,000 based on polyethylene glycol as measured by GPC under the below-mentioned conditions.

[Synthetic Example 3]

MPC (6.0 g) as monomer (1) and stearyl methacrylate (hereinafter referred to as "SMA") (3.3 g) as monomer (2a) (monomer (1)/monomer (2a)=80/20 (molar ratio)) were weighed into a glass flask for polymerization, ethanol (85.0 g) was added to dissolve monomers (1) and (2a), and azobisisobutyronitrile (0.06 g) as a polymerization initiator was added to the obtained solution. Thereafter, in the same manner as in Synthetic Example 1, a copolymer (hereinafter referred to as "polymer 3") was obtained. The weight average molecular weight of polymer 3 was 43,000 based on polyethylene glycol as measured by GPC under the below-mentioned conditions.

[Synthetic Example 4]

MPC (3.0 g) as monomer (1) and benzyl methacrylate (hereinafter referred to as "BzMA") (0.45 g) as monomer

(2c) (monomer (1)/monomer (2c)=80/20 (molar ratio)) were weighed into a glass flask for polymerization, ethanol (3.10 g) was added to dissolve monomers (1) and (2c), and azobisisobutyro nitrile (0.12 g) as a polymerization initiator was added to the obtained solution. Thereafter, in the same manner as in Synthetic Example 1, a copolymer (hereinafter referred to as "polymer 4") was obtained. The weight average molecular weight of polymer 4 was 240,000 based on polyethylene glycol as measured by GPC under the below-mentioned conditions.

[GPC measurement]

GPC measurement of the polymers 1 to 4 obtained in Synthetic Examples 1 to 4 was performed under the following conditions.

GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 μL

The monomers used in Synthetic Examples 1 to 4 and molar ratios thereof, and weight average molecular weights of the obtained polymers are summarized in Table 1.

[Table 1]

| | monomer (1) | monomer (2a) | monomer (2b) | monomer (2c) | molar ratio of monomers | weight average molecular weight |
|---|---|---|---|---|---|---|
| polymer 1 | MPC | MAc | | | 30/70/0/0 | 680,000 |
| polymer 2 | MPC | | PEGMA | | 90/0/10/0 | 501,000 |
| polymer 3 | MPC | SMA | | | 80/20/0/0 | 43,000 |
| polymer 4 | MPC | | | BzMA | 80/0/0/20 | 240,000 |

molar ratio of monomers = monomer (1)/monomer (2a)/monomer (2b)/monomer (2c)
MPC: 2-methacryloyloxyethyl phosphorylcholine
MAc: methacrylic acid
PEGMA: methoxypolyethylene glycol methacrylate
SMA: stearyl methacrylate
BzMA: benzyl methacrylate

[Examples 1 to 4]

Examples are shown in which the amplification target nucleic acid is a single-stranded RNA and the nucleic acid amplification method is an RT-qPCR method.

<Preparation of solution A>

Solution A was prepared by mixing the components listed in Table 2. This solution A was prepared while cooling on ice.

[Table 2]

| component | amount added *** |
|---|---|
| Reaction Buffer and dNTPs* | 8.00 μL |
| Manganese (II) Acetate* | 1.00 μL |

(continued)

| component | amount added *** |
|---|---|
| Primers and Probes No.2* | 1.00 μL |
| Hot Start Reverse Transcription DNA Polymerase* | 0.25 μL |
| 50x ROX** | 0.40 μL |
| Distilled Water, Nuclease-free** | 2.35 μL |
| total | 13.00 μL |
| * Components of SARS-CoV-2 RT-qPCR Detection Kit Ver.2 (manufactured by FUJIFILM Wako Pure Chemical Corporation)<br>** manufactured by Nippon Gene Co., Ltd.<br>*** amount added per 1 well | |

<Preparation of composition for nucleic acid amplification>

The polymers obtained in Synthetic Examples 1 to 4 were added to the obtained solution A to prepare compositions for nucleic acid amplification. In detail, 2 μL each of aqueous solutions of polymers 1 to 4 with a concentration of 0.5 w/v% were added to each well of solution A to prepare 15 μL of the compositions for nucleic acid amplification (concentration of polymer in each composition: 0.067 w/v%). These compositions were prepared while cooling on ice.

<Freeze-thawing of composition for nucleic acid amplification>

The obtained composition for nucleic acid amplification was placed in a PCR tube (manufactured by Eppendorf), and the tube was placed in an ultra-low temperature refrigerator (Deep Freezer manufactured by NIHON FREEZER CO., LTD.) and stored at - 80°C for 30 min to freeze the composition. The PCR tube containing the frozen composition was then placed in a refrigerator (manufactured by Sanyo Electric Co., Ltd.) and stored at 4°C for 2 hr to thaw the composition. This freeze-thawing operation was repeated 5 times.

The fluorescence intensity of the nucleic acid amplification product was measured as follows, using the composition immediately after preparation or the composition after five times of freeze-thawing.

<Preparation of RT-qPCR reaction solution>

An aqueous solution of the target nucleic acid with a nucleic acid concentration of 100 copy/μL was added to the composition for nucleic acid amplification in an amount of 5 μL per well to prepare a RT-qPCR reaction solution (concentration of each polymer in the RT-qPCR reaction solution: 0.050 w/v%). The PCR reaction solution was prepared while cooling on ice.

<RT-qPCR>

RT-qPCR was performed by a qPCR device ("StepOnePlus™ Real-Time PCR System" manufactured by Applied Biosystems), and fluorescence intensity at the endpoint of the PCR reaction liquid was measured. The reaction conditions (temperature program) thereof are as shown in the following Table 3.

[Table 3]

| step | temperature | time | cycle number |
|---|---|---|---|
| 1 | 90°C | 30 sec | |
| 2 | 60°C | 10 min | |
| 3 | 95°C | 1 min | |
| 4 | 95°C | 3 sec | 60 cycles |
| 5 | 60°C | 20 sec | |

Using the fluorescence intensity before freeze-thawing which was obtained using the composition immediately

after preparation and the fluorescence intensity after freeze-thawing which was obtained using the composition after 5 times of freeze-thawing, the relative fluorescence intensity was calculated by the following formula:

Relative fluorescence intensity (%) = 100 × fluorescence intensity after freeze-thawing/fluorescence intensity before freeze-thawing.

The results are shown in the following Table 4.

[Comparative Example 1]

The same operations as in Examples 1 to 4 were performed, except that 2 μL of Distilled Water, Nuclease-free (manufactured by Nippon Gene Co., Ltd.) was used instead of 2 μL of the aqueous solution of the polymer in <Preparation of composition for nucleic acid amplification>, and the relative fluorescence intensity was calculated. The results are shown in the following Table 4.

[Table 4]

| | protective agent used | relative fluorescence intensity (%) |
|---|---|---|
| Example 1 | polymer 1 | 81 |
| Example 2 | polymer 2 | 38 |
| Example 3 | polymer 3 | 56 |
| Example 4 | polymer 4 | 41 |
| Comparative Example 1 | - | 14 |

As shown in Table 4, Examples 1-4, which used polymers 1-4 as the protective agent, had higher relative fluorescence intensities than Comparative Example 1, which did not use a protective agent. In particular, the relative fluorescence intensity was 81% in Example 1. From these results, it is clear that the use of the protective agent of the present invention can reduce the deactivation of a composition for nucleic acid amplification due to freeze-thawing.

[Industrial Applicability]

The protective agent of the present invention can reduce the deactivation of a composition for nucleic acid amplification due to freeze-thawing. A composition for nucleic acid amplification containing the protective agent of the present invention is useful for nucleic acid amplification methods (particularly quantitative polymerase chain reaction method) for genetic tests, microbial tests, viral tests, and the like.

This application is based on a patent application No. filed in Japan 2023-055637, the contents of which are encompassed in full herein.

## Claims

1. A freeze-thawing protective agent for a composition for nucleic acid amplification, which is a polymer comprising constitutional units derived from a monomer represented by the formula (1):

[Chem. 1]

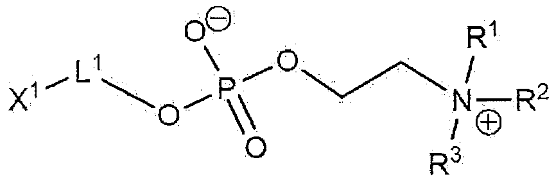

(1)

wherein

$X^1$ is a (meth)acryloyloxy group or a (meth)acryloylamino group,

$L^1$ is an alkylene group having 2 to 4 carbon atoms and optionally having one hydroxy group, or an alkyleneoxyalkylene group having 2 to 4 carbon atoms, and
$R^1$ to $R^3$ are each independently an alkyl group having 1 to 3 carbon atoms.

**2.** The freeze-thawing protective agent according to claim 1, wherein the polymer is a copolymer further comprising constitutional units derived from a monomer represented by the formula (2):

[Chem. 2]

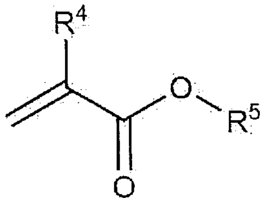

(2)

wherein

$R^4$ is a hydrogen atom or a methyl group, and
$R^5$ is a hydrogen atom, an alkyl group having 1 to 20 carbon atoms, $-(OCH_2CH_2)_nOR^6$ group (wherein n is a number from 1 to 10, and $R^6$ is a hydrogen atom, a methyl group, or an ethyl group), or an aralkyl group having 7 to 12 carbon atoms.

**3.** A composition for nucleic acid amplification, comprising the freeze-thawing protective agent according to claim 2 and water.

**4.** The composition according to claim 3, further comprising a polymerase.

**5.** The composition according to claim 4, further comprising a primer.

**6.** The composition according to any one of claims 3 to 5, which is frozen.

**7.** A method for amplifying a nucleic acid, comprising preparing a reaction solution for nucleic acid amplification by mixing the composition according to any one of claims 3 to 5 and a sample comprising the nucleic acid to be amplified.

**8.** A method for amplifying a nucleic acid, comprising preparing a reaction solution for nucleic acid amplification by mixing a composition for nucleic acid amplification obtained by thawing the frozen composition for nucleic acid amplification according to claim 6 and a sample comprising the nucleic acid to be amplified.

**INTERNATIONAL SEARCH REPORT**

International application No.
**PCT/JP2024/010666**

**A. CLASSIFICATION OF SUBJECT MATTER**

***C12Q 1/6844***(2018.01)i; ***C08F 20/36***(2006.01)i; ***C12Q 1/48***(2006.01)i
FI: C12Q1/6844 Z; C08F20/36; C12Q1/48 Z

According to International Patent Classification (IPC) or to both national classification and IPC

**B. FIELDS SEARCHED**

Minimum documentation searched (classification system followed by classification symbols)

C12Q1/6844; C08F20/36; C12Q1/48

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2022/163507 A1 (NOF CORPORATON) 04 August 2022 (2022-08-04) claims 1-7, paragraphs [0007], [0011], [0020]-[0023], [0042], [0043], [0076]-[0079], examples, in particular, example 9 | 3-5, 7 |
| Y | claims 1-7, paragraphs [0007], [0011], [0020]-[0023], [0042], [0043], [0076]-[0079], examples, in particular, example 9 | 1-8 |
| X | US 2019/0360021 A1 (NANOHELIX CO., LTD.) 28 November 2019 (2019-11-28) claims 1-13, paragraphs [0014]-[0016], [0031]-[0038], examples | 3-5, 7 |
| Y | claims 1-13, paragraphs [0014]-[0016], [0031]-[0038], examples | 1-8 |
| Y | WO 2018/216628 A1 (NOF CORPORATON) 29 November 2018 (2018-11-29) claims 1-4, paragraphs [0018], [0036]-[0038], [0041], examples | 1-8 |
| Y | JP 10-045794 A (NOF CORPORATION) 17 February 1998 (1998-02-17) claims 1-3, paragraph [0006], examples | 1-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/010666**

C. DOCUMENTS CONSIDERED TO BE RELEVANT

| Category* | Citation of document, with indication, where appropriate, of the relevant passages | Relevant to claim No. |
|---|---|---|
| P, X | WO 2023/054340 A1 (NOF CORPORATON) 06 April 2023 (2023-04-06) claims 1-3, examples, in particular, paragraphs [0097], [0107], [0113]-[0115] | 3-5, 7 |
| P, Y | claims 1-3, examples, in particular, paragraphs [0097], [0107], [0113]-[0115] | 1-8 |

Form PCT/ISA/210 (second sheet) (July 2022)

**INTERNATIONAL SEARCH REPORT**
**Information on patent family members**

International application No.
**PCT/JP2024/010666**

| Patent document cited in search report | Publication date (day/month/year) | Patent family member(s) | Publication date (day/month/year) |
|---|---|---|---|
| WO 2022/163507 A1 | 04 August 2022 | EP 4286533 A1<br>claims, examples, paragraph [0008], examples 9<br>CN 116888275 A<br>KR 10-2023-0137403 A | |
| US 2019/0360021 A1 | 28 November 2019 | WO 2019/103164 A1<br>claim 1-13, examples<br>KR 10-2019-0088559 A | |
| WO 2018/216628 A1 | 29 November 2018 | US 2020/0102415 A1<br>claims, examples, paragraphs [0019], [0037]-[0039]. [0042]<br>CN 110650969 A | |
| JP 10-045794 A | 17 February 1998 | (Family: none) | |
| WO 2023/054340 A1 | 06 April 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008203269 A
- JP 2017137328 A
- WO 2018216628 A
- JP 2023055637 A